# EUROPÄISCHE PATENTSCHRIFT

(11) **EP 2 755 938 B1**
(45) Veröffentlichungstag und Bekanntmachung des Hinweises auf die Patenterteilung: **01.03.2017**
(21) Anmeldenummer: 12756224.7
(22) Anmeldetag: 10.09.2012
(51) Int. Cl.: C07C 29/19, C07C 29/44, C07C 31/135, C07C 33/26, B01J 37/18, B01J 21/08, B01J 23/46, B01J 23/56, B01J 33/00, B01J 35/00, B01J 35/02, B01J 35/08, B01J 35/10, B01J 37/02, C07C 29/80

(54) **VERFAHREN ZUR HERSTELLUNG VON 4-CYCLOHEXYL-2-METHYL-2-BUTANOL**
METHOD FOR PRODUCING 4-CYCLOHEXYL-2-METHYL-2-BUTANOL
PROCÉDÉ DE PRÉPARATION DE 4-CYCLOHEXYL-2-MÉTHYL-2-BUTANOL

(30) Priorität: 16.09.2011 EP 11181599
(43) Veröffentlichungstag der Anmeldung: 23.07.2014
(73) Patentinhaber: BASF SE, 67056 Ludwigshafen am Rhein (DE)
(72) Erfinder: SCHUCH, Rudolf, 69198 Schriesheim (DE); RÜDENAUER, Stefan, 67549 Worms (DE); EBEL, Klaus, 68623 Lampertheim (DE); PELZER, Ralf, 37699 Fürstenberg (DE); KELLER, Andreas, 67346 Speyer (DE)
(86) Internationale Anmeldenummer: PCT/EP2012/067655
(87) Internationale Veröffentlichungsnummer: WO 2013/037737

(56) Entgegenhaltungen:
- US-A- 4 701 278
- NAKAGAWA T ET AL: "Reactions of supercritical alcohols with unsaturated hydrocarbons", JOURNAL OF SUPERCRITICAL FLUIDS, PRA PRESS, US, Bd. 27, Nr. 3, 1. Dezember 2003 (2003-12-01), Seiten 255-261, XP004458590, ISSN: 0896-8446, DOI: 10.1016/S0896-8446(02)00269-3 in der Anmeldung erwähnt
- KAMITANAKA ET AL: "Direct addition of supercritical alcohols, acetone or acetonitrile to the alkenes without catalysts", TETRAHEDRON LETTERS, ELSEVIER, AMSTERDAM, NL, Bd. 48, Nr. 48, 31. Oktober 2007 (2007-10-31), Seiten 8460-8463, XP022322876, ISSN: 0040-4039, DOI: 10.1016/J.TETLET.2007.09.159 in der Anmeldung erwähnt
- OKAZAWA ET AL: "solution carbocation stabilities measured by internal competition for a hydride ion", CANADIAN JOURNAL OF CHEMISTRY, NRC RESEARCH PRESS, CA, Bd. 60, 1. Januar 1982 (1982-01-01), Seiten 2180-2193, XP002257156, ISSN: 0008-4042, DOI: 10.1139/V82-311 in der Anmeldung erwähnt

## Beschreibung

Die vorliegende Erfindung betrifft ein Verfahren zur Herstellung von 4-Cyclohexyl-2-methyl-2-butanol.

4-Cyclohexyl-2-methyl-2-butanol, das auch als Coranol bezeichnet wird, ist ein Riechstoff mit einem Maiglöckchenduft, dessen Verwendung als Bestandteil von Duftzusammensetzungen erstmalig in US 4,701,278 beschrieben wurde.

Die Herstellung von 4-Cyclohexyl-2-methyl-2-butanol wurde von N.E. Okazawa et al. in Can. J. Chem. 60 (1982), 2180-93 beschrieben und umfasst die Umwandlung von 3-Cyclohexylpropansäure in ihr Säurechlorid, das anschließend mit 2 mol Methyllithium zum 4-Cyclohexyl-2-methyl-2-butanol umgesetzt wird. Aufgrund der Verwendung von Methyllithium ist dieses Herstellungsverfahren, insbesondere bei der Durchführung in größerem Maßstab, mit nicht unbeträchtlichen Risiken behaftet und wirtschaftlich unattraktiv. Dennoch wurden bislang in der Literatur keine weiteren Herstellungsverfahren beschrieben.

Die unveröffentlichte Anmeldung PCT/EP2011/054559 der Anmelderin beschreibt ein Verfahren zur Herstellung von 4-Cyclohexyl-2-methyl-2-butanol. Das dort beschriebene Verfahren offenbart die Umsetzung von Styrol mit Isopropanol bei erhöhter Temperatur. Die Beispiele 1 bis 4 offenbaren eine Umsetzung von Stryol mit Isopropanol, wobei das Isopropanol batchweise zur Reaktion zugegeben wird. Der Umsatz in den Beispielen 1 bis 4 beträgt immer über 85%, so dass die molaren Verhältnisse von Stryol zu Isopropanol in den Beispielen 1 bis 4 immer deutlich über 1: 5 liegen.

Der vorliegenden Erfindung liegt daher die Aufgabe zugrunde, ein wirtschaftliches Verfahren zur Herstellung von 4-Cyclohexyl-2-methyl-2-butanol bereit zu stellen. Diese Aufgabe wird durch das im Folgenden beschriebene Verfahren gelöst, welches die folgenden Schritte umfasst:
a) Umsetzung von Styrol mit Isopropanol bei einer Temperatur im Bereich von 250 bis 500 °C und unter Bedingungen, bei denen Isopropanol im überkritischen Zustand vorliegt, wobei man 2-Methyl-4-phenyl-2-butanol erhält, und
b) heterogen-katalytische Hydrierung von 2-Methyl-4-phenyl-2-butanol an einem zur Kernhydrierung von Aromaten geeigneten Kontakt,
wobei das Molverhältnis des in Schritt a) eingesetzten Styrols zu dem in Schritt a) eingesetzten Isopropanol im Bereich von 1 : unter 5 bis 1 : 0,5 liegt.

Das Verfahren lässt sich durch das folgende Reaktionsschema darstellen:

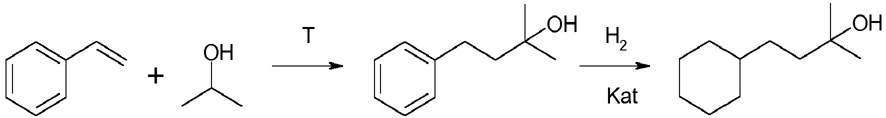

Die Erfindung betrifft somit ein Verfahren zur Herstellung von 4-Cyclohexyl-2-methyl-2-butanol mit dem hier und im Folgenden und in den Ansprüchen beschriebenen Schritten.

Das Verfahren ist mit einer Reihe von Vorteilen verbunden. Es erlaubt die Herstellung von 4-Cyclohexyl-2-methyl-2-butanol aus sehr preiswerten Grundchemikalien. Der Einsatz teurer und gefährlicher Reagenzien wie Methyllithium ist nicht erforderlich. Sowohl Schritt a) als auch Schritt b) lassen sich in technischem Maßstab problemlos durchführen und liefern die jeweiligen Produkte mit hoher Selektivität und guten Ausbeuten.

In Schritt a) des erfindungsgemäßen Verfahrens wird Isopropanol mit Styrol bei erhöhter Temperatur umgesetzt. Hierbei bildet sich im Sinne einer Hydroxyalkylierung 2-Methyl-4-phenyl-2-butanol sowie als Nebenprodukte unter anderem Toluol und Ethylbenzol, die jedoch vom Zielprodukt beispielsweise durch Destillation abgetrennt werden können.

Über die Umsetzung wurde von T. Nakagawa et al. im Rahmen einer analytischen Untersuchung zur Umsetzung von Styrol mit Alkanolen unter überkritischen Bedingungen berichtet (siehe J. Supercritical Fluids, 27 (2003), S. 255-261 und Tetrahedron Lett., 48 (2007), S. 8460-8463). Die Reaktion wurde jedoch bislang nicht zur präparativen Gewinnung von 2-Methyl-4-phenyl-2-butanol genutzt.

Im Hinblick auf die Selektivität der Reaktion hat es sich als vorteilhaft erwiesen, wenn man Umsetzung in Schritt a) unter überkritischen Bedingungen durchführt. Hierunter versteht man Reaktionsbedingungen, bei denen wenigstens eine der Komponenten der Reaktionsmischung, vorzugsweise das Isopropanol, im überkritischen Zustand vorliegt. Dementsprechend erfolgt die Umsetzung in Schritt a) unter Bedingungen, bei denen Isopropanol im überkritischen Zustand vorliegt. Die kritische Temperatur T_{c} von Isopropanol ist 235 °C, der kritische Druck P_{c} beträgt 4,8 MPa. Überkritische Bedingungen können vom Fachmann durch Variation von Druck und Temperatur eingestellt werden.

Die für eine hinreichende Geschwindigkeit der Umsetzung von Styrol mit Isopropanol erforderliche Temperatur beträgt in der Regel wenigstens 250 °C, häufig wenigstens 300 °C und insbesondere wenigstens 320 °C. Zur Erzielung einer hinreichenden Selektivität der Reaktion hat es sich als vorteilhaft erwiesen, wenn die Temperatur der Umsetzung einen Wert von 500 °C nicht überschreitet. Die Umsetzung gemäß Schritt a) erfolgt vorzugsweise bei erhöhtem Druck, der in der Regel im Bereich von 5 bis 50 MPa, häufig im Bereich von 10 bis 30 MPa und insbesondere im Bereich von 15 bis 25 MPa. Der Druck im Reaktionsgefäß kann durch Beaufschlagung mit einer Inerten eingestellt werden. Vorzugsweise erfolgt die Umsetzung unter dem bei der gewünschten Reaktionstemperatur herrschenden Eigendruck der Reaktionsmischung.

Die Reaktionsdauer hängt naturgemäß von den gewählten Bedingungen und dem gewünschten Umsatz ab und liegt üblicherweise im Bereich von 30 sec. bis 4 h, insbesondere im Bereich von 3 min. bis 3 h und speziell im Bereich von 5 min bis 2,5 h. In der Regel wird die Reaktion soweit geführt, dass der im Unterschuss eingesetzte Reaktand, bei dem es sich in der vorzugsweise um Styrol handelt, zu wenigstens 80 %, insbesondere zu wenigstens 90 % umgesetzt ist.

In einer Ausführungsform der Erfindung liegt die Reaktionsdauer im Bereich von 30 min. bis 4 h, insbesondere im Bereich von 1 bis 3 h und speziell im Bereich von 1,5 bis 2,5 h. In der Regel wird die Reaktion soweit geführt, dass der im Unterschuss eingesetzte Reaktand, bei dem es sich in der vorzugsweise um Styrol handelt, zu wenigstens 80 %, insbesondere zu wenigstens 90 % umgesetzt ist.

Als besonders vorteilhaft hat es sich erwiesen, die Umsetzung des Schritts a) bei erhöhten Temperaturen, d.h. oberhalb 300°C, insbesondere oberhalb 320°C, insbesondere im Bereich von 350°C und 500°C, vorzugsweise im Bereich von 390°C und 500°C durchzuführen. Dies erlaubt kurze Reaktionszeiten, die üblicherweise im Bereich von 30 sec. bis 30 min, insbesondere im Bereich von 3 min bis 20 min und speziell im Bereich von 5 min bis 15 min liegen. Auf diese Weise können auch bei hohem Umsatz an Styrol Selektivitäten bezüglich des Zielproduktes von deutlich > 60 % erzielt werden.

Im Hinblick auf die Selektivität der Reaktion hat es sich als vorteilhaft erwiesen, wenn man die Umsetzung gemäß Schritt a) in weitgehender oder vollständiger Abwesenheit von Katalysatoren, wie beispielsweise Radikalstarter, Säuren oder Übergangsmetallverbindungen durchführt. Weitgehende Abwesenheit bedeutet, dass die Konzentration etwaige Katalysatoren weniger als 1 g/kg (< 1000 ppm), insbesondere weniger 0,1 g/kg (< 100 ppm) bezogen auf das Gesamtgewicht der Reaktionsmischung, beträgt.

Die Umsetzung von Styrol mit Isopropanol gemäß Schritt a) kann in Substanz oder in einem geeigneten, d. h. unter Reaktionsbedingungen inerten, Verdünnungsmittel durchgeführt werden. Geeignete inerte Verdünnungsmittel sind aprotische organische Lösungsmittel, die keine ethylenisch ungesättigte Doppelbindung aufweisen, wie beispielsweise aliphatische und alicyclische Ether mit vorzugsweise 4, 5 oder 6 C-Atomen, aliphatische und cycloaliphatische gesättigte Kohlenwasserstoffe mit vorzugsweise 6 bis 8 C-Atomen, Alkylester aliphatischer Carbonsäuren mit vorzugsweise 4 bis 8 C-Atomen und Gemische der vorgenannten Lösungsmittel. Vorzugsweise erfolgt die Umsetzung gemäß Schritt a) in Substanz, d. h. zur Umsetzung werden im Wesentlichen keine von Styrol und Isopropanol verschiedenen Einsatzstoffe, wie beispielsweise inerte Lösungsmittel, eingesetzt. Im Wesentlichen bedeutet hier, dass Styrol und Isopropanol wenigstens 95 Gew.-%, insbesondere wenigstens 99 Gew.-%, bezogen auf die Gesamtmenge der in Schritt a) eingesetzten Komponenten, ausmachen. Daneben können die zur Reaktion eingesetzten Reaktanden, d. h. Styrol und Isopropanol, herstellungsbedingt geringe Mengen an Verunreinigungen wie Wasser, Ethylbenzol, Toluol und dergleichen enthalten, wobei die Verunreinigungen in der Regel weniger als 5 Gew.-%, insbesondere weniger als 1 Gew.-%, bezogen auf die Gesamtmenge der Reaktanden ausmachen. Insbesondere beträgt der Wassergehalt der in Schritt a) eingesetzten Reaktanden nicht mehr als 1 Gew.-%, bezogen auf die Gesamtmenge der Reaktanden.

Im erfindungsgemäßen Verfahren setzt man in Schritt a) Styrol und Isopropanol in einem Molverhältnis Styrol zu Isopropanol ein wobei das Molverhältnis des in Schritt a) eingesetzten Styrol zu dem in Schritt a) eingesetzten Isopropanol im Bereich von 1 : unter 5 bis 1 : 0,5 liegt.

Im Hinblick auf eine effiziente Reaktionsführung ist es von Vorteil, wenn man in Schritt a) Styrol und Isopropanol in einem Molverhältnis im Bereich von 1 : 4,9 bis 1 : 0,5, insbesondere 1 : 4,5 bis 1 : 0,5, vorzugsweise im Bereich von 1 : 4 bis 1 : 0,75, insbesondere im Bereich von 1 : 3,5 bis 1 : 1,5 oder im Bereich von 1:2 bis 1:1, vorzugsweise im Bereich von 1 : 1 einsetzt.

Die Umsetzung in Schritt a) kann absatzweise durchgeführt werden (so genannte batch-Fahrweise), d. h. Styrol und Isopropanol werden im gewünschten Molverhältnis in einem geeigneten Reaktor vorgelegt und auf die gewünschten Reaktionsbedingungen gebracht und bis zu dem gewünschten Umsatz unter Reaktionsbedingungen gehalten. Die Umsetzung in Schritt a) kann auch in der so genannte semi-batch-Fahrweise durchgeführt werden, d. h. die Hauptmenge, in der Regel wenigstens 80 %, einer oder beide Reaktanden, wird kontinuierlich oder portionsweise über einen längeren Zeitraum, in der Regel wenigstens 50 % der gesamten Umsatzdauer, unter Reaktionsbedingungen in den Reaktor gegeben. Die Umsetzung in Schritt a) kann auch kontinuierlich durchgeführt werden, d. h. Styrol und Isopropanol werden im gewünschten Molverhältnis kontinuierlich in eine Reaktionszone eingespeist und das Reaktionsgemisch wird kontinuierlich der Reaktionszone entnommen. Die Rate, mit der Styrol und Isopropanol der Reaktionszone zugeführt werden, richtet sich nach der gewünschten Verweilzeit, die ihrerseits in bekannter Weise von der Reaktorgeometrie abhängt und der oben angegebenen Umsetzungsdauer entspricht.

Die Umsetzung gemäß Schritt a) kann grundsätzlich in allen Reaktoren durchgeführt werden, die für die gewählten Reaktionsbedingungen geeignet sind, vorzugsweise in Autoklaven, die Vorrichtungen zur Durchmischung der Reaktanden aufweisen können, oder in Reaktionsrohren.

Um das Molverhältnis von Styrol zu Isopropanol während der Umsetzung niedrig zu halten und gleichzeitig eine effiziente Reaktionsführung zu erlauben, hat es sich als vorteilhaft erwiesen, wenn man wenigstens 80 %, insbesondere wenigstens 90 % des in Schritt a) eingesetzten Isopropanols gegebenenfalls zusammen mit einer Teilmenge des Styrols vorlegt und wenigstens 80 %, insbesondere wenigstens 90 % des in Schritt a) eingesetzten Styrols der Umsetzung in Schritt a) unter Reaktionsbedingungen zuführt. Die Zugabe des Styrols kann portionsweise oder vorzugsweise kontinuierlich erfolgen. Die Geschwindigkeit, mit der man Styrol zuführt wird dabei vorzugweise so gewählt, dass das Molverhältnis des in die Reaktionszone bzw. den Reaktor eingespeisten, noch nicht abreagierten Styrols zu dem in der Reaktionszone befindlichen Isopropanol während der Umsetzung weniger als 1 : unter 5 beträgt, insbesondere nicht mehr 1: 4,9, insbesondere nicht mehr als 1 : 4,5 und speziell nicht mehr als 1 : 4 beträgt, z. B. im Bereich von 1 : 4,9 bis 1 : 0,5, insbesondere 1 : 4,5 bis 1 : 0,5, vorzugsweise im Bereich von 1 : 4 bis 1 : 0,75 und insbesondere im Bereich von 1 : 3,5 bis 1 : 1,5 liegt. Bei einer kontinuierlichen Reaktionsführung wird man daher vorzugsweise Styrol und Isopropanol in den zuvor genannten Molverhältnissen dem Reaktor bzw. der Reaktionszone zuführen. In einer Ausführungsform der Erfindung wird die Geschwindigkeit, mit der man Styrol zuführt vorzugweise so gewählt, dass das Molverhältnis des in die Reaktionszone bzw. den Reaktor eingespeisten, Styrols zu dem in der Reaktionszone befindlichen Isopropanol im Bereich von 1: unter 5, insbesondere im Bereich von 1: 4,9 bis 1 : 0,5 , insbesondere 1 : 4,5 bis 1 : 0,5, besonders bevorzugt im Bereich von 1 : 4 bis 1 : 0,75 und speziell im Bereich von 1 : 3,5 bis 1 : 1,5 insbesondere 1 : 2 bis 1 : 1 liegt. Dies gilt insbesondere auch bei kontinuierlicher Reaktionsführung für die dem Reaktor bzw. der Reaktionszone zugeführten Molverhältnisse von Styrol und Isopropanol.

Ein weiterer Gegenstand der vorliegenden Erfindung betrifft ein Verfahren zur Herstellung von 2-Methyl-4-phenyl-2-butanol, umfassend a) die Umsetzung von Styrol mit Isopropanol bei einer Temperatur im Bereich von 250 bis 500 °C und unter Bedingungen, bei denen Isopropanol im überkritischen Zustand vorliegt, wobei das Molverhältnis des in Schritt a) eingesetzten Styrols zu dem in Schritt a) eingesetzten Isopropanol im Bereich von 1 : unter 5 bis 1 : 0,5 liegt.

Als besonders vorteilhaft hat es sich erwiesen, die Umsetzung des Schritts a) bei erhöhten Temperaturen, d.h. oberhalb 300°C, insbesondere oberhalb 320°C, insbesondere im Bereich von 350°C und 500°C, vorzugsweise im Bereich von 390°C und 500°C durchzuführen.

Eine bevorzugten Ausführungsform der Erfindung betrifft eine Verfahren zur Herstellung von 2-Methyl-4-phenyl-2-butanol, umfassend a) die Umsetzung von Styrol mit Isopropanol bei einer Temperatur im Bereich von 250 bis 500 °C und unter Bedingungen, bei denen Isopropanol im überkritischen Zustand vorliegt, wobei das Molverhältnis des in Schritt a) eingesetzten Styrols zu dem in Schritt a) eingesetzten Isopropanol im Bereich von 1 : unter 5 bis 1 : 0,5 liegt und wobei die Temperaturen größer gleich 390 °C, insbesondere größer gleich 450 °C beträgt.

Im Übrigen gelten für diesen weiteren Gegenstand der Erfindung die oben unter Schritt a) genannten bevorzugten Reaktionsbedingungen.

Überraschenderweise wurde gefunden, dass mit dem erfindungsgemäßen Verfahren im Vergleich zum Stand der Technik auch deutlich größere Mengenverhältnisse von Styrol zu Isopropanol angewendet werden können, ohne dass größere Umsatz- und Ausbeuteverluste auftreten. Dies wird auch dadurch ermöglicht, dass mit dem erfindungsgemäßen Verfahren eine unerwünschte Bildung von Polystyrol reduziert wird - somit steht mehr Styrol-Monomer der Reaktion zur Verfügung und Verlegungen des Reaktionssystemes werden verhindert.

Das in Schritt a) erhaltene Reaktionsgemisch kann in an sich bekannter Weise aufgearbeitet werden oder unmittelbar als solches in Schritt b) des erfindungsgemäßen Verfahrens eingesetzt werden. In der Regel hat es sich als vorteilhaft erwiesen, das in Schritt a) anfallende Reaktionsgemisch aufzuarbeiten, beispielsweise extraktive oder destillativ oder durch eine Kombination dieser Maßnahmen. In einer Ausführungsform des erfindungsgemäßen Verfahrens arbeitet man das in Schritt a) anfallende Reaktionsgemisch destillativ auf, wobei man das gewünschte 2-Methyl-4-phenyl-2-butanol als Mittelfraktion von Leicht- und Schwersiedern abtrennt. Sofern mit einem Isopropanol-Überschuss gearbeitet wird, kann das die Leichtsieder-Fraktion, die überwiegend aus Isopropanol besteht, in den Prozess zurückgeführt werden. In der Regel wird man vor Schritt b) Isopropanol weitgehend entfernen, so dass der Anteil an Isopropanol in dem zur Hydrierung in Schritt b) eingesetzten Edukt weniger als 20 Gew.-%, insbesondere nicht mehr als 10 Gew.-%, bezogen auf die Gesamtmenge an Edukt in Schritt b), beträgt.

Je nach Ausgestaltung der Destillation erhält man ein reines Methyl-4-phenyl-2-butanol (Reinheit ≥ 95 Gew.-%, insbesondere ≥ 98 Gew.-% und speziell ≥ 99 Gew.-% oder ≥ 99,5 Gew.-%) oder eine Zusammensetzung, die im wesentlichen, d.h. zu wenigstens 95 Gew.-%, insbesondere wenigstens 98 Gew.-% und speziell wenigstens 99 Gew.-% oder wenigstens 99,5 Gew.-% aus 2-Methyl-4-phenyl-2-butanol und geringen Mengen an 2-Methyl-4-phenyl-2-pentanol, besteht, z.B. Zusammensetzungen, worin das Gewichtsverhältnis von 2-Methyl-4-phenyl-2-butanol zu 2-Methyl-4-phenyl-2-pentanol im Bereich von 50:1 bis 1000:1 liegt. Sowohl das reine 2-Methyl-4-phenyl-2-butanol als auch die Zusammensetzung, die im wesentlichen aus 2-Methyl-4-phenyl-2-butanol und geringen Mengen an 2-Methyl-4-phenyl-2-pentanol besteht können in der anschließenden Hydrierung gemäß Schritt b) eingesetzt werden und liefern entsprechend reines 4-Cyclohexyl-2-methyl-2-butanol (Reinheit ≥ 95 Gew.-%, insbesondere ≥ 98 Gew.-% und speziell ≥ 99 Gew.-% oder ≥ 99,5 Gew.-%) oder eine Zusammensetzung, die im wesentlichen, d.h. zu wenigstens 95 Gew.-%, insbesondere wenigstens 98 Gew.-% und speziell wenigstens 99 Gew.-% oder wenigstens 99,5 Gew.-% aus 4-Cyclohexyl-2-methyl-2-butanol und geringen Mengen an 4-Cyclohexyl-2-methyl-2-pentanol, besteht, z.B. Zusammensetzungen, worin das Gewichtsverhältnis von 4-Cyclohexyl-2-methyl-2-butanol zu 4-Cyclohexyl-2-methyl-2-pentanol im Bereich von 50:1 bis 1000:1 liegt.

Das in Schritt a) gewonnene 2-Methyl-4-phenyl-2-butanol wird anschließend in Schritt b) des erfindungsgemäßen Verfahrens einer heterogen-katalytischen Hydrierung an einem zur Kernhydrierung von Aromaten geeigneten Katalysator, der im Folgenden auch als Kontakt bezeichnet wird, unterworfen.

Geeignete Kontakte sind grundsätzlich alle Kontakte, die bekanntermaßen zur Kernhydrierung von Aromaten geeignet sind, d. h. Kontakte, welche die Hydrierung von Phenylgruppen zu Cyclohexylgruppen katalysieren. Hierbei handelt es sich üblicherweise um Kontakte, die wenigstens ein Aktivmetall aus der Gruppe VIIIB des Periodensystems (CAS-Version) enthalten, wie z. B. Palladium, Platin, Eisen, Cobalt, Nickel, Rhodium, Iridium, Ruthenium, insbesondere Ruthenium, Rhodium oder Nickel, oder ein Gemisch aus zwei oder mehr davon enthalten, gegebenenfalls in Kombination mit einem oder mehreren weiteren Aktiv-Metallen. Bevorzugte weitere Aktivmetalle sind aus den Gruppen IB oder VIIB des Periodensystems (CAS-Version) ausgewählt. Unter den ebenfalls verwendbaren Metallen der Nebengruppen IB und/oder VIIB des Periodensystems der Elemente sind z. B. Kupfer und/oder Rhenium geeignet.

Bei den Katalysatoren kann es sich um Vollkatalysatoren oder vorzugsweise um Trägerkatalysatoren handeln. Geeignete Trägermaterialien sind beispielsweise Aktivkohle, Siliciumcarbid, Siliciumdioxid, Aluminiumoxid, Magnesiumoxid, Titandioxid, Zirkondioxid, Alumosilicate und Gemische dieser Trägermaterialien. Die Menge an Aktivmetall beträgt üblicherweise 0,05 bis 10 Gew.-%, häufig 0,1 bis 7 Gew.-% und insbesondere 4 bis 7 Gew.-%, vorzugsweise 5 bis 7 Gew.% bezogen auf das Gesamtgewicht des Trägerkatalysators, insbesondere, wenn es sich bei dem Aktivmetall um ein Edelmetall wie Rhodium, Ruthenium, Platin, Palladium oder Iridium handelt. Bei Katalysatoren, die als Aktivmetalle Eisen und/oder Cobalt und/oder Nickel enthalten, kann die Menge an Aktivmetall bis zu 100 Gew.-% betragen und liegt üblicherweise im Bereich von 1 bis 100 Gew.-%, insbesondere 10 bis 90 Gew.-% bezogen auf das Gesamtgewicht des Katalysators.

Die Trägerkatalysatoren können in Form eines Pulvers eingesetzt werden. In der Regel weist ein solches Pulver Teilchengrößen im Bereich von 1 bis 200 µm insbesondere 1 bis 100 µm auf. Pulverförmige Katalysatoren eignen sich insbesondere dann, wenn der Katalysator in der zu hydrierenden Reaktionsmischung suspendiert wird (Suspensionsfahrweise). Bei Einsatz des Katalysatoren in Katalysatorfestbetten verwendet man üblicherweise Formkörper, die z. B. die Form von Kugeln, Tabletten, Zylindern, Strängen, Ringen bzw. Hohlzylindern, Sternen und dergleichen aufweisen können. Die Abmessungen dieser Formkörper bewegen sich üblicherweise im Bereich von 0,5 mm bis 25 mm. Häufig werden Katalysatorstränge mit Strangdurchmessern von 1,0 bis 5 mm und Stranglängen von 2 bis 25 mm eingesetzt. Mit kleineren Strängen können im Allgemeinen höhere Aktivitäten erzielt werden; jedoch zeigen diese oft keine ausreichende mechanische Stabilität im Hydrierverfahren. Daher werden ganz besonders bevorzugt Stränge mit Strangdurchmessern im Bereich von 1,5 bis 3 mm eingesetzt. Ebenfalls bevorzugt sind Kugelförmige Trägermaterialien mit Kugeldurchmessern im Bereich von 1 bis 10 mm, insbesondere 2 bis 6 mm.

Bevorzugte Kontakte sind solche, die wenigstens ein unter Ruthenium, Rhodium und Nickel ausgewähltes Aktivmetall und gegebenenfalls in Kombination mit einem oder mehreren weiteren Aktivmetallen, die aus den Gruppen IB, VIIB oder VIIIB des Periodensystems (CAS-Version) ausgewählt sind.

Besonders bevorzugte Kontakte sind Ruthenium-haltige Kontakte. Diese enthalten Ruthenium als Aktivmetall, gegebenenfalls in Kombination mit einem oder mehreren weiteren Aktivmetallen. Bevorzugte weitere Aktivmetalle sind aus den Gruppen IB, VIIB oder VIIIB des Periodensystems (CAS-Version) ausgewählt. Bei den Katalysatoren kann es sich um Vollkatalysatoren oder vorzugsweise um Trägerkatalysatoren handeln. Beispiele für weitere Aktivmetalle aus der Gruppe VIIIB sind z. B. Platin, Rhodium, Palladium, Iridium, Eisen, Cobalt oder Nickel oder ein Gemisch aus zwei oder mehr davon. Unter den ebenfalls verwendbaren Metallen der Nebengruppen IB und/oder VIIB des Periodensystems der Elemente sind z. B. Kupfer und/oder Rhenium geeignet. Bevorzugt wird Ruthenium alleine als Aktivmetall oder zusammen mit Platin oder Iridium als Aktivmetall eingesetzt; ganz besonders bevorzugt wird Ruthenium alleine als Aktivmetall eingesetzt.

Bevorzugt sind insbesondere Ruthenium-haltige Kontakte, in den das Ruthenium auf einem Trägermaterial angeordnet ist, sog. Ruthenium-haltige Trägerkatalysatoren. Die Trägermaterialien derartiger Trägerkatalysatoren weisen in der Regel eine spezifischen BET-Oberfläche, bestimmt durch N₂-Adsorbtion nach DIN 66131,von wenigstens 30 m²/g, insbesondere 50 bis 1500 m²/g, vorzugsweise 800 bis 1200 m²/g auf. Bevorzugt sind Siliziumdioxid-haltige Trägermaterialien, insbesondere solche, die einen Gehalt an Siliziumdioxid von wenigstens 90 Gew.-%, bezogen auf das Gesamtgewicht des Trägermaterials aufweisen. Ebenfalls bevorzugt sind Aluminiumoxid-haltige Trägermaterialien, insbesondere solche, die einen Gehalt an Aluminiumoxid (gerechnet als Al₂O₃) von wenigstens 90 Gew.-%, bezogen auf das Gesamtgewicht des Trägermaterials aufweisen. Ebenfalls bevorzugt sind Aktivkohle-haltige Trägermaterialien, diese sind beispielsweise kommerziell erhältlich unter dem Handelsnamen "Norit SX plus" der Fa. Norit.

Geeignete Ruthenium-haltige Kontakte sind beispielsweise die in US 3027398, DE 4407091, EP 258789, EP 813906, EP 1420012, WO 99/32427, WO 00/78704, WO 02/100536, WO 03/103830, WO 2005/61105, WO 2005/61106, WO 2006/136541, EP 1317959 sowie die in der nicht vorveröffentlichten EP 09179201.0 genannten Kontakte. Bezüglich der darin offenbarten Katalysatoren wird auf diese Schriften Bezug genommen.

Ebenfalls bevorzugte Kontakte sind Rhodium-haltige Kontakte. Diese enthalten Rhodium als Aktivmetall, gegebenenfalls in Kombination mit einem oder mehreren weiteren Aktivmetallen. Bevorzugte weitere Aktivmetalle sind aus den Gruppen IB, VIIB oder VIIIB des Periodensystems (CAS-Version) ausgewählt. Bei den Katalysatoren kann es sich um Vollkatalysatoren oder vorzugsweise um Trägerkatalysatoren handeln. Beispiele für weitere Aktivmetalle sind aus der Gruppe VIIIB sind z. B. Platin, Palladium, Iridium, Eisen, Cobalt oder Nickel oder ein Gemisch aus zwei oder mehr davon. Unter den ebenfalls verwendbaren Metallen der Nebengruppen IB und/oder VIIB des Periodensystems der Elemente sind z. B. Kupfer und/oder Rhenium geeignet. In diesen Katalysatoren wird vorzugsweise Rhodium alleine als Aktivmetall eingesetzt. Geeignete Rhodium-haltige Kontakte sind beispielsweise aus den zuvor für Ruthenium-haltige Katalysatoren genannten Publikationen bekannt oder können nach den dort angegebenen Vorgehensweisen hergestellt werden oder sind kommerziell erhältlich, z.B. der Katalysator Escat 34 der Fa. Engelhard.

Ebenfalls bevorzugte Kontakte sind Nickel-haltige Kontakte. Diese enthalten Nickel als Aktivmetall, gegebenenfalls in Kombination mit einem oder mehreren weiteren Aktivmetallen. Bevorzugte weitere Aktivmetalle sind aus den Gruppen IB, VIIB oder VIIIB des Periodensystems (CAS-Version) ausgewählt. Bei den Katalysatoren kann es sich um Vollkatalysatoren oder vorzugsweise um Trägerkatalysatoren handeln. Beispiele für weitere Aktivmetalle sind aus der Gruppe VIIIB sind z. B. Platin, Palladium, Iridium, Eisen oder Cobalt oder ein Gemisch aus zwei oder mehr davon. Unter den ebenfalls verwendbaren Metallen der Nebengruppen IB und/oder VIIB des Periodensystems der Elemente sind z. B. Kupfer und/oder Rhenium geeignet. In diesen Katalysatoren wird vorzugsweise Nickel alleine als Aktivmetall eingesetzt. Geeignete Nickel-haltige Kontakte sind kommerziell erhältlich, beispielsweise BASF Katalysator Ni5249P.

Besonders bevorzugt wird in Schritt b) als Kontakt ein Trägerkatalysator eingesetzt, der als Aktivmetall Ruthenium alleine oder zusammen mit mindestens einem weiteren Aktivmetall der Nebengruppen IB, VIIB oder VIIIB des Periodensystems der Elemente (CAS-Version) auf einen Trägermaterial enthält. Bevorzugt wird Ruthenium alleine als Aktivmetall oder zusammen mit Eisen, Platin oder Iridium als Aktivmetall eingesetzt; ganz besonders bevorzugt wird Ruthenium alleine oder in Kombination mit Eisen als Aktivmetall eingesetzt. Als Trägermaterialien für die Ruthenium-haltigen Trägerkatalysatoren kommen grundsätzlich die vorgenannten Trägermaterialien in Betracht. Bevorzugt sind Siliziumdioxid-haltige Trägermaterialien, insbesondere solche, die einen Gehalt an Siliziumdioxid von wenigstens 90 Gew.-%, bezogen auf das Gesamtgewicht des Trägermaterials aufweisen. Ebenfalls bevorzugt sind Aluminiumoxid-haltige Trägermaterialien, insbesondere solche, die einen Gehalt an Aluminiumoxid (gerechnet als Al₂O₃) von wenigstens 90 Gew.-%, bezogen auf das Gesamtgewicht des Trägermaterials aufweisen. Bevorzugt sind Trägermaterialien mit einer spezifischen BET-Oberfläche, bestimmt durch N₂-Adsorbtion nach DIN 66131von wenigstens 30 m²/g, insbesondere 50 bis 1500 m²/g, insbesondere 800 bis 1200 m²/g. Die Menge an Aktivmetall beträgt üblicherweise 0,05 bis 10 Gew.-%, vorzugsweise 0,1 bis 3 Gew.-% und insbesondere 0,1 bis 1 Gew.-%, bezogen auf das Gesamtgewicht des Ruthenium-haltigen Trägerkatalysators.. Ebenfalls bevorzugt sind Aktivkohle-haltige Trägermaterialien, diese sind beispielsweise kommerziell erhältlich unter dem Handelsnamen "Norit SX plus" der Fa. Norit.

Ebenfalls bevorzugt wird in Schritt b) als Kontakt ein Trägerkatalysator eingesetzt, der als Aktivmetall Rhodium alleine oder zusammen mit mindestens einem weiteren Aktivmetall der Nebengruppen IB, VIIB oder VIIIB des Periodensystems der Elemente (CAS-Version) auf einen Trägermaterial enthält. Bevorzugt wird Rhodium alleine als Aktivmetall oder zusammen mit Platin oder Iridium als Aktivmetall eingesetzt; ganz besonders bevorzugt wird Rhodium alleine als Aktivmetall eingesetzt. Als Trägermaterialien für die Rhodium-haltigen Trägerkatalysatoren kommen grundsätzlich die vorgenannten Trägermaterialien in Betracht. Bevorzugt sind Siliziumdioxid-haltige Trägermaterialien, insbesondere solche, die einen Gehalt an Siliziumdioxid von wenigstens 90 Gew.-%, bezogen auf das Gesamtgewicht des Trägermaterials aufweisen. Ebenfalls bevorzugt sind Aluminiumoxid-haltige Trägermaterialien, insbesondere solche, die einen Gehalt an Aluminiumoxid (gerechnet als Al₂O₃) von wenigstens 90 Gew.-%, bezogen auf das Gesamtgewicht des Trägermaterials aufweisen. Die Menge an Aktivmetall beträgt üblicherweise 0,05 bis 10 Gew.-%, bezogen auf das Gesamtgewicht des Rhodium-haltigen Trägerkatalysators.

Ebenfalls bevorzugt wird in Schritt b) als Kontakt ein Katalysator eingesetzt, der als Aktivmetall Nickel alleine oder zusammen mit mindestens einem weiteren Aktivmetall der Nebengruppen IB, VIIB oder VIIIB des Periodensystems der Elemente (CAS-Version), gegebenenfalls auf einen Trägermaterial enthält. Bevorzugt wird Nickel alleine als Aktivmetall eingesetzt. Als Trägermaterialien für die Nickel-haltigen Trägerkatalysatoren kommen grundsätzlich die vorgenannten Trägermaterialien in Betracht. Bevorzugt sind Siliciumdioxid, Aluminiumoxid und Magnesiumoxid-haltige Trägermaterialen, insbesondere solche, die zu wenigstens 90 Gew.-% aus derartigen Materialien bestehen. Die Menge an Aktivmetall beträgt üblicherweise 1 bis 90 Gew.-% vorzugsweise 10 bis 80 Gew.-% und insbesondere 30 bis 70 Gew.-%, bezogen auf das Gesamtgewicht des Nickel-haltigen Trägerkatalysators. Bevorzugt sind auch solche Nickel-haltigen Kontakte, die im Wesentlichen ausschließlich aus Aktiv-Metall bestehen, d.h. deren Menge an Aktivmetall mehr als 90 Gew.-%, z.B. 90 bis 100 Gew.-% beträgt.

Gemäß einer besonders bevorzugten Ausführungsform wird ein Schalenkatalysator eingesetzt, insbesondere ein Schalenkatalysator, welcher als Aktivmetall Ruthenium alleine oder zusammen mit mindestens einem weiteren Aktivmetall der Nebengruppen IB, VIIB oder VIIIB des Periodensystems der Elemente in den oben genannten Mengen aufweist. Derartige Schalenkatalysatoren sind insbesondere aus der WO 2006/136541 bekannt.

Ein solcher Schalenkatalysator ist ein Trägerkatalysator, der dadurch gekennzeichnet ist, dass sich die überwiegende Menge des bzw. der im Katalysator enthaltenen Aktivmetalle sich in der Nähe der Oberfläche des Katalysators befinden. Insbesondere liegen mindestens 60 Gew.-% besonders bevorzugt mindestens 80 Gew.-%, jeweils bezogen auf die Gesamtmenge des Aktivmetalls, bis zu einer Eindringtiefe von maximal 200 µm, d. h. in einer Schale mit einem Abstand von maximal 200 µm zur Oberfläche der Katalysatorpartikel, vor. Dagegen liegt im Inneren (Kern) des Katalysators keine oder nur eine sehr geringe Menge des Aktivmetalls vor. Ganz besonders bevorzugt ist ein erfindungsgemäßer Schalenkatalysator, in dem kein Aktivmetall im Inneren des Katalysators nachgewiesen werden kann, d. h. Aktivmetall liegt nur in der äußersten Schale, zum Beispiel in einer Zone bis zu einer Eindringtiefe von 100 bis 200 µm, vor. Die vorstehend genannten Daten können mittels SEM (scanning electron microscopy), EPMA (electron probe microanalysis) - EDXS (energy dispersive X-ray spectroscopy) ermittelt werden und stellen gemittelte Werte dar. Weitere Angaben bezüglich den vorstehend genannten Messverfahren und Techniken können zum Beispiel "Spectroscopy in Catalysis" von J. W. Niemantsverdriet, VCH, 1995 entnommen werden. Wegen weiterer Details bezüglich der Eindringtiefe an Aktivmetall wird auf die WO 2006/136541, insbesondere auf S. 7, Zeilen 6 bis 12 verwiesen.

Bevorzugte Schalenkatalysatoren weisen einen Gehalt an Aktivmetall im Bereich von 0,05 bis 1 Gew.%, insbesondere 0,1 bis 0,5 Gew.-%, besonders bevorzugt 0,25 bis 0,35 Gew.-%, jeweils bezogen auf das Gesamtgewicht des Katalysators auf.

Für die erfindungsgemäße Hydrierung in Schritt b) sind Schalenkatalysatoren mit einem Trägermaterial auf Basis von Siliziumdioxid, im Allgemeinen amorphem Siliziumdioxid besonders bevorzugt. Unter dem Begriff "amorph" versteht man in diesem Zusammenhang, dass der Anteil kristalliner Siliziumdioxid-Phasen weniger als 10 Gew.-% des Trägermaterials ausmacht. Die zur Herstellung der Katalysatoren verwendeten Trägermaterialien können allerdings Überstrukturen aufweisen, die durch regelmäßige Anordnung von Poren im Trägermaterial gebildet werden. Als Trägermaterialien kommen grundsätzlich amorphe Siliziumdioxid-Typen in Betracht, die wenigstens zu 90 Gew.-% aus Siliziumdioxid bestehen, wobei die verbleibenden 10 Gew.-%, vorzugsweise nicht mehr als 5 Gew.-%, des Trägermaterials auch ein anderes oxidisches Material sein können, z. B. MgO, CaO, TiO₂, ZrO₂, Fe₂O₃ und/oder Alkalimetalloxid. In einer bevorzugten Ausführungsform des Schalenkatalysators ist das Trägermaterial halogenfrei, insbesondere chlorfrei, d. h. der Gehalt an Halogen im Trägermaterial beträgt weniger als 500 Gew.-ppm, z. B. im Bereich von 0 bis 400 Gew.-ppm. Somit ist ein Schalenkatalysator bevorzugt, der weniger als 0,05 Gew.-% Halogenid (ionen-chromatographisch bestimmt), bezogen auf das Gesamtgewicht des Katalysators, enthält. Bevorzugt sind Trägermaterialien, die eine spezifische Oberfläche im Bereich von 30 bis 700 m²/g, vorzugsweise 30 bis 450 m²/g, (BET-Oberfläche nach DIN 66131) aufweisen. Geeignete amorphe Trägermaterialien auf Basis von Siliziumdioxid sind dem Fachmann geläufig und kommerziell erhältlich (siehe z. B. O. W. Flörke, "Silica" in Ullmann's Encyclopedia of Industrial Chemistry, 6th Edition on CD-ROM). Sie können sowohl natürlichen Ursprungs als auch künstlich hergestellt worden sein. Beispiele für geeignete amorphe Trägermaterialien auf Basis von Siliziumdioxid sind Kieselgele, Kieselgur, pyrogene Kieselsäuren und Fällungskieselsäuren. In einer bevorzugten Ausführungsform der Erfindung weisen die Katalysatoren Kieselgele als Trägermaterialien auf. Je nach Ausgestaltung des Schalenkatalysators kann das Trägermaterial unterschiedliche Gestalt aufweisen. Sofern das Verfahren, in dem die erfindungsgemäßen Schalenkatalysatoren eingesetzt werden, als Suspensionsverfahren ausgestaltet ist, wird man zur Herstellung der erfindungsgemäßen Katalysatoren üblicherweise das Trägermaterial in Form eines feinteiligen Pulvers einsetzen. Vorzugsweise weist das Pulver Teilchengrößen im Bereich von 1 bis 200 µm insbesondere 1 bis 100 µm auf. Bei Einsatz des erfindungsgemäßen Schalenkatalysators in Katalysatorfestbetten verwendet man üblicherweise Formkörper aus dem Trägermaterial, die z. B. durch Extrudieren, Strangpressen oder Tablettieren erhältlich sind und die z. B. die Form von Kugeln, Tabletten, Zylindern, Strängen, Ringen bzw. Hohlzylindern, Sternen und dergleichen aufweisen können. Die Abmessungen dieser Formkörper bewegen sich üblicherweise im Bereich von 0,5 mm bis 25 mm. Häufig werden Katalysatorstränge mit Strangdurchmessern von 1,0 bis 5 mm und Stranglängen von 2 bis 25 mm eingesetzt. Mit kleineren Strängen können im Allgemeinen höhere Aktivitäten erzielt werden; jedoch zeigen diese oft keine ausreichende mechanische Stabilität im Hydrierverfahren. Daher werden ganz besonders bevorzugt Stränge mit Strangdurchmessern im Bereich von 1,5 bis 3 mm eingesetzt. Ebenfalls bevorzugt sind Kugelförmige Trägermaterialien mit Kugeldurchmessern im Bereich von 1 bis 10 mm, insbesondere 2 bis 6 mm.

In einer besonders bevorzugten Ausführungsform der Schalenkatalysatoren weist das Trägermaterial des Katalysators, bei dem es sich insbesondere um ein Trägermaterial auf Basis von Siliziumdioxid handelt, ein Porenvolumen im Bereich von 0,6 bis 1,0 ml/g, bevorzugt im Bereich von 0,65 bis 0,9 ml/g, beispielsweise 0,7 bis 0,8 ml/g, bestimmt durch Hg-Porosimetrie (DIN 66133), und eine BET-Oberfläche im Bereich 280 bis 500 m²/g, vorzugsweise im Bereich von 280 bis 400 m²/g, ganz besonders bevorzugt im Bereich von 300 bis 350 m²/g auf. Vorzugsweise weisen in derartigen Schalenkatalysatoren mindestens 90 % der vorhandenen Poren einen Durchmesser von 6 bis 12 nm, bevorzugt 7 bis 11 nm, besonders bevorzugt 8 bis 10 nm, auf. Der Porendurchmesser kann nach dem Fachmann bekannten Verfahren bestimmt werden, beispielsweise durch Hg-Porosimetrie oder N₂-Physisorption. In einer bevorzugten Ausführungsform weisen mindestens 95 %, besonders bevorzugt mindestens 98 %, der vorhandenen Poren einen Porendurchmesser von 6 bis 12 nm, bevorzugt 7 bis 11 nm, besonders bevorzugt 8 bis 10 nm, auf. In einer bevorzugten Ausführungsform liegen in diesen Schalenkatalysatoren keine Poren vor, die kleiner als 5 nm sind. Des Weiteren liegen in diesen Schalenkatalysatoren vorzugsweise keine Poren vor, die größer als 25 nm, insbesondere größer als 15 nm, sind. In diesem Zusammenhang bedeutet "keine Poren", dass mit üblichen Messverfahren, beispielsweise Hg-Porosimetrie oder N₂-Physisorption keine Poren mit diesen Durchmessern gefunden werden.

In bevorzugten Schalenkatalysatoren beträgt die Dispersität des Aktivmetalls bevorzugt 30 bis 60 %, besonders bevorzugt 30 bis 50 %. Verfahren zur Messung der Dispersität des Aktivmetalls sind dem Fachmann an sich bekannt, beispielsweise durch Pulschemisorption, wobei die Bestimmung der Edelmetalldispersion (spezifische Metalloberfläche, Kristallitgröße) mit CO Pulsmethode durchgeführt wird (DIN 66136(1-3)).

Das erfindungsgemäße Hydrierverfahren kann in der Flüssigphase oder in der Gasphase durchgeführt werden. Bevorzugt wird das erfindungsgemäße Hydrierverfahren in der Flüssigphase durchgeführt.

Das erfindungsgemäße Hydrierverfahren kann in Abwesenheit eines Lösungs- oder Verdünnungsmittels oder Gegenwart eines Lösungs- oder Verdünnungsmittels durchgeführt werden, d. h. es ist nicht erforderlich, die Hydrierung in Lösung durchzuführen. Als Lösungs- oder Verdünnungsmittel kann jedes geeignete Lösungsmittel oder Verdünnungsmittel eingesetzt werden. Als Lösungs- oder Verdünnungsmittel kommen grundsätzlich solche in Betracht, die die zu hydrierende organische Verbindung möglichst vollständig zu lösen vermögen oder sich mit dieser vollständig mischen und die unter den Hydrierungsbedingungen inert sind, d. h. nicht hydriert werden. Beispiele für geeignete Lösungsmittel sind cyclische und acyclische Ether mit vorzugsweise 4 bis 8 C-Atomen, z. B. Tetrahydrofuran, Dioxan, Methyl-tert.-butylether, Dimethoxyethan, Dimethoxypropan, Dimethyldiethylenglykol, aliphatische Alkohole mit vorzugsweise 1 bis 6 C-Atomen wie Methanol, Ethanol, n- oder Isopropanol, n-, 2-, iso- oder tert.-Butanol, Carbonsäureester aliphatischer Carbonsäuren mit vorzugsweise 3 bis 8 C-Atomen wie Essigsäuremethylester, Essigsäureethylester, Essigsäurepropylester oder Essigsäurebutylester, Propionsäuremethylester, Propionsäureethylester, Propionsäurebutylester sowie aliphatische Etheralkohole wie Methoxypropanol und cycloaliphatische Verbindungen wie Cyclohexan, Methylcyclohexan und Dimethylcyclohexan. Die Menge des eingesetzten Lösungs- oder Verdünnungsmittels ist nicht in besonderer Weise beschränkt und kann je nach Bedarf frei gewählt werden, wobei bei Verwendung eines Lösungsmittels jedoch solche Mengen bevorzugt sind, die zu einer 3 bis 70 gew.-%igen Lösung der zur Hydrierung vorgesehenen organischen Verbindung führen.

In einer Ausführungsform der Erfindung wird der erfindungsgemäße Schritt b) in Substanz durchgeführt.

Die eigentliche Hydrierung erfolgt üblicherweise in Analogie zu den bekannten Hydrierverfahren zur Hydrierung von organischen Verbindungen, die hydrierbare Gruppen aufweisen, bevorzugt zur Hydrierung einer carbocyclischen aromatischen Gruppe zu der entsprechenden carbocyclischen aliphatischen Gruppe, wie sie im eingangs genannten Stand der Technik beschrieben werden. Hierzu wird die organische Verbindung als flüssige Phase oder Gasphase, bevorzugt als flüssige Phase, mit dem Katalysator in Gegenwart von Wasserstoff in Kontakt gebracht. Die flüssige Phase kann man über ein Katalysator-Fließbett (Fließbett-Fahrweise) oder ein Katalysator-Festbett (Festbettfahrweise) geleitet werden.

Die Hydrierung kann sowohl kontinuierlich als auch diskontinuierlich ausgestaltet werden, wobei die kontinuierliche Verfahrensdurchführung bevorzugt ist. Vorzugsweise führt man das erfindungsgemäße Verfahren in Rieselreaktoren oder in gefluteter Fahrweise nach der Festbettfahrweise durch, wobei die Durchführung in Rieselreaktoren besonders bevorzugt ist. Insbesondere wird hier die zu hydrierende Verbindung in Substanz, d. h. weitgehender Abwesenheit organischer Verdünnungsmittel eingesetzt (Lösungsmittelgehalt vorzugsweise < 10 %). Der Wasserstoff kann dabei sowohl im Gleichstrom mit der Lösung des zu hydrierenden Edukts als auch im Gegenstrom über den Katalysator geleitet werden. Die Hydrierung kann auch diskontinuierlich nach der Batchfahrweise durchgeführt werden. In diesem Fall wird man die Hydrierung vorzugsweise in einem organischen Lösungs- oder Verdünnungsmittel durchführen.

In einer weiteren Ausführungsform der Erfindung kann die Hydrierung in Suspensionsfahrweise durchgeführt werden, insbesondere in kontinuierlicher Suspensionsfahrweise.

Bei einer diskontinuierlichen Durchführung des erfindungsgemäßen Verfahrens wird in Schritt b) der Katalysator typischerweise in einer Menge eingesetzt, dass die Konzentration an Ruthenium in dem zur Hydrierung eingesetzten Reaktionsansatz im Bereich von 10 bis 10000 ppm, insbesondere im Bereich von 50 bis 5000 ppm, speziell im Bereich von 100 bis 1000 ppm liegt.

Die Hydrierung erfolgt typischerweise bei einem Wasserstoffdruck im Bereich von 5 bis 50 MPa, insbesondere im Bereich von 10 bis 30 MPa. Der Wasserstoff kann als solcher, oder verdünnt mit einer Inerte, beispielsweise Stickstoff oder Argon in den Reaktor eingespeist werden.

Die Hydrierung in Schritt b) erfolgt typischerweise bei Temperaturen oberhalb 50 °C, insbesondere im Bereich von 100 bis 250 °C.

Zur Durchführung der Hydrierung geeignete Apparaturen sind dem Fachmann bekannt und richten sich in erster Linie nach der Fahrweise. Geeignete Apparaturen zur Durchführung einer Hydrierung nach der Hydrierung am Katalysatorfließbett und am Katalysatorfestbett sind z. B. aus Ullmanns Enzyklopädie der Technischen Chemie, 4. Auflage, Band 13, S. 135 ff., sowie aus P. N. Rylander, "Hydrogenation and Dehydrogenation" in Ullmann's Encyclopedia of Industrial Chemistry, 5th ed. on CD-ROM, bekannt.

Es wurde überraschenderweise gefunden, dass Schritt a) des erfindungsgemäßen Verfahrens neben 2-Methyl-4-phenyl-2-butanol auch 2-Methyl-4-phenyl-2-pentanol liefert, wenn man es unter Bedingungen durchführt, bei denen Isopropanol unter überkritischen Bedingungen vorliegt, insbesondere wenn das Molverhältnis von Styrol zu Isopropanol im Bereich von 1 : unter 5 bis 1 : 0,5, insbesondere im Bereich von 1: 4,9 bis 1: 0,5, insbesondere 1 : 4,5 bis 1 : 0,5, besonders bevorzugt im Bereich von 1 : 4 bis 1 : 0,75 und speziell im Bereich von 1 : 3,5 bis 1 : 1,5 insbesondere 1 : 2 bis 1: 1 liegt.
In einer bevorzugten Ausführungsform wird das erfindungsgemäße Verfahren in semi-batch-Fahrweise durchgeführt.

Ein solches Verfahren ist neu und ebenfalls Gegenstand der vorliegenden Erfindung. Dementsprechend betrifft die Erfindung ein Verfahren zur Herstellung einer Zusammensetzung, enthaltend 2-Methyl-4-phenyl-2-butanol und 2-Methyl-4-phenyl-2-pentanol, umfassend die Umsetzung von Styrol mit Isopropanol bei erhöhter Temperatur unter Bedingungen, bei denen Isopropanol unter überkritischen Bedingungen vorliegt, wobei das Molverhältnis von Styrol zu Isopropanol im Bereich von 1 : unter 10, vorzugsweise 1: 9 bis 1: 0,5, vorzugsweise 1: 5 bis 1 : 0,5, insbesondere im Bereich von 1 : 4,5 bis 1 : 0,5, besonders bevorzugt im Bereich von 1 : 4 bis 1 : 0,75 und speziell im Bereich von 1 : 3,5 bis 1 : 1,5 insbesondere 1 : 2 bis 1 : 1 liegt.

Dabei hat es sich als vorteilhaft erwiesen, wenn man die Umsetzung in semi-batch-Fahrweise oder kontinuierlich, wie zuvor bereits für Schritt a) beschrieben, durchführt. Hierfür hat es sich insbesondere als vorteilhaft erwiesen, wenn man wenigstens 80 %, insbesondere wenigstens 90 % des eingesetzten Isopropanols gegebenenfalls zusammen mit einer Teilmenge des Styrols vorlegt und wenigstens 80 %, insbesondere wenigstens 90 % des eingesetzten Styrols der Umsetzung unter Reaktionsbedingungen zuführt. Die Zugabe des Styrols kann portionsweise oder vorzugsweise kontinuierlich erfolgen. Die Geschwindigkeit, mit der man Styrol zuführt wird dabei vorzugweise so gewählt, dass das Molverhältnis des in die Reaktionszone bzw. den Reaktor eingespeisten, Styrols zu dem in der Reaktionszone befindlichen Isopropanol im Bereich von 1 : unter 10, vorzugsweise 1 : 9 bis 1 : 0,5, vorzugsweise 1 : 5 bis 1 : 0,5, insbesondere im Bereich von 1 : 4,5 bis 1 : 0,5, besonders bevorzugt im Bereich von 1 : 4 bis 1 : 0,75 und speziell im Bereich von 1 : 3,5 bis 1 : 1,5 insbesondere 1 : 2 bis 1 : 1 liegt. Dies gilt insbesondere auch bei kontinuierlicher Reaktionsführung für die dem Reaktor bzw. der Reaktionszone zugeführten Molverhältnisse von Styrol und Isopropanol.

Im Übrigen gelten die zuvor für Schritt a) genannten Bedingungen in gleicher Weise auch für das erfindungsgemäße Verfahren zur Herstellung einer Zusammensetzung, enthaltend 2-Methyl-4-phenyl-2-butanol und 2-Methyl-4-phenyl-2-pentanol. Auf diese Angaben wird daher vollumfänglich Bezug genommen.

Ein derartiges Verfahren liefert Zusammensetzungen, die 2-Methyl-4-phenyl-2-butanol und 2-Methyl-4-phenyl-2-pentanol enthalten. Das Gewichtsverhältnis von 2-Methyl-4-phenyl-2-butanol zu 2-Methyl-4-phenyl-2-pentanol liegt in derartigen Zusammensetzungen typischerweise im Bereich von 50:1 bis 1000:1.

Das bei der Herstellung einer Zusammensetzung, enthaltend 2-Methyl-4-phenyl-2-butanol und 2-Methyl-4-phenyl-2-pentanol, durch Umsetzung von Styrol mit Isopropanol anfallende Reaktionsgemisch kann in der zuvor für Schritt a) beschriebenen Weise aufgearbeitet werden. Auf die zuvor gemachten Angaben zur Aufarbeitung des in Schritt a) anfallenden Reaktionsgemischs wird vollumfänglich Bezug genommen. Insbesondere arbeitet man das anfallende Reaktionsgemisch destillativ auf, wobei man die gewünschte Zusammensetzung, bestehend im wesentlichen aus 2-Methyl-4-phenyl-2-butanol und 2-Methyl-4-phenyl-2-pentanol als Mittelfraktion von Leicht- und Schwersiedern abtrennt. In der Regel erhält man dann eine Zusammensetzung, die im wesentlichen, d.h. zu wenigstens 95 Gew.-%, insbesondere wenigstens 98 Gew.-% und speziell wenigstens 99 Gew.-% oder wenigstens 99,5 Gew.-% aus 2-Methyl-4-phenyl-2-butanol und geringen Mengen an 2-Methyl-4-phenyl-2-pentanol, besteht, z.B. Zusammensetzungen, worin das Gewichtsverhältnis von 2-Methyl-4-phenyl-2-butanol zu 2-Methyl-4-phenyl-2-pentanol im Bereich von 50:1 bis 1000:1 liegt.

Derartige Gemische aus 2-Methyl-4-phenyl-2-butanol und geringen Mengen an 2-Methyl-4-phenyl-2-pentanol können anschließend in Analogie zu Schritt b) hydriert werden, wobei man Zusammensetzungen erhält, die 4-Cyclohexyl-2-methyl-2-butanol und geringe Mengen an 4-Cyclohexyl-2-methyl-2-pentanol, enthalten, z.B. Zusammensetzungen, worin das Gewichtsverhältnis von 4-Cyclohexyl-2-methyl-2-butanol zu 4-Cyclohexyl-2-methyl-2-pentanol im Bereich von 50:1 bis 1000:1 liegt. Auf die zuvor gemachten Angaben zur Hydrierung in Schritt b) wird vollumfänglich Bezug genommen. Da in einer Ausführungsform der Erfindung bei der Herstellung einer Zusammensetzung, enthaltend 2-Methyl-4-phenyl-2-butanol und 2-Methyl-4-phenyl-2-pentanol mit einem Isopropanol-Überschuss gearbeitet wird, kann in dieser Ausführungsform die Leichtsieder-Fraktion, die überwiegend aus Isopropanol besteht, in den Prozess zurückgeführt werden. In der Regel wird man vor der Hydrierung Isopropanol weitgehend entfernen, so dass der Anteil an Isopropanol in dem zur Hydrierung eingesetzten Edukt weniger als 20 Gew.-%, insbesondere nicht mehr als 10 Gew.-%, bezogen auf die Gesamtmenge an Edukt, beträgt.

Zusammensetzungen, enthaltend 2-Methyl-4-phenyl-2-butanol und 2-Methyl-4-phenyl-2-pentanol zeichnen sich überraschenderweise dadurch aus, dass sie im Vergleich zum 2-Methyl-4-phenyl-2-butanol eine blumigere Geruchsnote aufweisen. In diesen Zusammensetzungen liegt das Gewichtsverhältnis von 2-Methyl-4-phenyl-2-butanol zu 2-Methyl-4-phenyl-2-pentanol im Bereich von 50:1 bis 1000:1. Eine spezielle Zusammensetzung sind Konzentrate, d.h. Zusammensetzungen, die im wesentlichen, d.h. zu wenigstens 95 Gew.-%, insbesondere wenigstens 98 Gew.-% und speziell wenigstens 99 Gew.-% oder wenigstens 99,5 Gew.-% aus 2-Methyl-4-phenyl-2-butanol und geringen Mengen an 2-Methyl-4-phenyl-2-pentanol, besteht, z.B. Zusammensetzungen, worin das Gewichtsverhältnis von 2-Methyl-4-phenyl-2-butanol zu 2-Methyl-4-phenyl-2-pentanol im Bereich von 50:1 bis 1000:1 liegt. Zusammensetzungen, in denen das Gewichtsverhältnis von 2-Methyl-4-phenyl-2-butanol zu 2-Methyl-4-phenyl-2-pentanol außerhalb der hier genannten Bereich liegt, können durch Vermischen von 2-Methyl-4-phenyl-2-butanol (Muguet-Alkohol) mit der gewünschten Menge an 2-Methyl-4-phenyl-2-pentanol hergestellt werden.

Derartige Zusammensetzungen, insbesondere die vorgenannten Konzentrate, können aus den zuvor genannten Gründen als Duft- bzw. Aromastoffe verwendet werden, insbesondere in kosmetischen Zusammensetzungen sowie in Wasch- oder Reinigungszusammensetzungen.

Im Rahmen dieser Erfindung wurde weiterhin gefunden, dass 2-Methyl-4-phenyl-2-pentanol sich auch gezielt dadurch herstellen lässt, dass man α-Methylstyrol mit Isopropanol unter den zuvor für Schritt a) genannten Bedingungen umsetzt.

Gegenstand der Erfindung ist daher auch ein Verfahren zur Herstellung von 2-Methyl-4-phenyl-2-pentanol, bei dem α-Methylstyrol mit Isopropanol bei einer Temperatur im Bereich von 250 bis 500 °C und unter Bedingungen, bei denen Isopropanol im überkritischen Zustand vorliegt, umgesetzt wird, wobei das Molverhältnis des eingesetzten α-Methylstyrol zum eingesetzten Isopropanol im Bereich von 1 : unter 5 bis 1 : 0,5 liegt, vorzugsweise in Bereich von 1 : 4,9 bis 1 : 0,5, insbesondere im Bereich von 1 : 4,5 bis 1 : 0,5, besonders bevorzugt im Bereich von 1 : 4 bis 1 : 0,75 und speziell im Bereich von 1 : 3,5 bis 1 : 1,5 insbesondere 1 : 2 bis 1 : 1 liegt.

Für die Umsetzung von α-Methylstyrol mit Isopropanol gelten im Wesentlichen alle Angaben, die zuvor und in den Ansprüchen für die Umsetzung von Styrol mit Isopropanol gemäß Schritt a) gemacht wurden. Auf diese Angaben wird daher vollumfänglich Bezug genommen.

Alternativ kann 2-Methyl-4-phenyl-2-pentanol oder ein Gemisch, das im wesentlichen aus 2-Methyl-4-phenyl-2-pentanol besteht, durch destillative Trennung von Gemischen, die 2-Methyl-4-phenyl-2-butanol und 2-Methyl-4-phenyl-2-pentanol enthalten, z.B. durch destillative Trennung von Gemischen, wie sie in Schritt a) des erfindungsgemäßen Verfahrens anfallen, gewonnen werden.

2-Methyl-4-phenyl-2-pentanol ist ebenfalls ein Geruchsstoff und kann daher in allen Anwendungen für Geruchsstoffe dieses Typs verwendet werden. Wie bereits zuvor gesagt, kann es überraschenderweise auch zur Modifizierung der Geruchseigenschaften von 2-Methyl-4-phenyl-2-butanol verwendet werden.

2-Methyl-4-phenyl-2-pentanol kann in Analogie zu Schritt b) einer Hydrierung unterworfen werden. Hierbei erhält man in hoher Ausbeute 4-Cyclohexyl-2-methyl-2-pentanol.

4-Cyclohexyl-2-methylpentanol ist, ähnlich 4-Cyclohexyl-2-methyl-2-butanol, ein Geruchsstoff. Zudem kann es überraschenderweise zur Modifizierung der Geruchseigenschaften von anderen Geruchsstoffen, insbesondere von 4-Cyclohexyl-2-methyl-2-butanol verwendet werden. 4-Cyclohexyl-2-methyl-2-pentanol kann daher als Duft- bzw. Aromastoff verwendet werden, insbesondere in kosmetischen Zusammensetzungen sowie in Wasch- oder Reinigungszusammensetzungen.

In diesen Zusammensetzungen liegt das Gewichtsverhältnis von 4-Cyclohexyl-2-methyl-2-butanol zu 4-Cyclohexyl-2-methyl-2-pentanol in der Regel im Bereich von 50:1 bis 1000:1. Derartige Zusammensetzungen können auch geringe Mengen an 4-Cyclohexyl-2-methylbutan und gegebenenfalls 4-Cyclohexyl-2-methylpentan enthalten, die durch Überreduktion von 2-Methyl-4-phenyl-2-butanol bzw. 2-Methyl-4-phenyl-2-pentanol erhalten. Der Gewichtsanteil der Gesamtmenge an Cyclohexyl-2-methylbutan und gegebenenfalls 4-Cyclohexyl-2-methylpentan wird in der Regel 10 Gew.-%, insbesondere 5 Gew.-%, bezogen auf 4-Cyclohexyl-2-methyl-2-butanol, nicht überschreiten und liegt, sofern vorhanden im Bereich von 0,01 bis 10 Gew.-%, insbesondere im Bereich von 0,01 bis 5 Gew.-%, bezogen auf 4-Cyclohexyl-2-methyl-2-butanol. Es ist selbstverständlich auch möglich, Cyclohexyl-2-methylbutan und gegebenenfalls 4-Cyclohexyl-2-methylpentan abzutrennen, z.B. auf destillativem Wege, so dass die Gesamtmenge an Cyclohexyl-2-methylbutan und gegebenenfalls 4-Cyclohexyl-2-methylpentan weniger als 1 Gew.-%, insbesondere weniger als 0,5 Gew.-% oder weniger als 0,1 Gew.-%, bezogen auf 4-Cyclohexyl-2-methyl-2-butanol, beträgt. Eine spezielle Zusammensetzung sind Konzentrate, d.h. Zusammensetzungen, die im wesentlichen, d.h. zu wenigstens 95 Gew.-%, insbesondere wenigstens 98 Gew.-% und speziell wenigstens 99 Gew.-% oder wenigstens 99,5 Gew.-% aus 2-4-Cyclohexyl-2-methyl-2-butanol und geringen Mengen an 4-Cyclohexyl-2-methyl-2-pentanol besteht, z.B. Zusammensetzungen, worin das Gewichtsverhältnis von 4-Cyclohexyl-2-methyl-2-butanol zu 4-Cyclohexyl-2-methyl-2-pentanol im Bereich von 50:1 bis 1000:1 liegt.

Diese Konzentrate können Cyclohexyl-2-methylbutan und gegebenenfalls 4-Cyclohexyl-2-methylpentan in den oben genannten Mengen enthalten. Zusammensetzungen, in denen das Gewichtsverhältnis von 4-Cyclohexyl-2-methyl-2-butanol zu 4-Cyclohexyl-2-methyl-2-pentanol außerhalb der hier genannten Bereich liegt, können durch Vermischen von Cyclohexyl-2-methyl-2-butanol mit der gewünschten Menge an 4-Cyclohexyl-2-methyl-2-pentanol hergestellt werden.

Derartige Zusammensetzungen, insbesondere die vorgenannten Konzentrate, können aus den zuvor genannten Gründen als Duft- bzw. Aromastoffe verwendet werden, insbesondere in kosmetischen Zusammensetzungen sowie in Wasch- oder Reinigungszusammensetzungen.

Wie bereits zuvor gesagt, kann 4-Cyclohexyl-2-methyl-2-pentanol aus 2-Methyl-4-phenyl-2-pentanol in Analogie zu Schritt b) hergestellt werden, d.h. durch ein Verfahren, umfassend eine heterogen-katalytische Hydrierung von 2-Methyl-4-phenyl-2-pentanol an einem zur Kernhydrierung von Aromaten geeigneten Kontakt. Ein solches Verfahren ist ebenfalls Gegenstand der vorliegenden Erfindung. Auf die zuvor gemachten Angaben zur Hydrierung in Schritt b) wird bezüglich der Hydrierung von 2-Methyl-4-phenyl-2-pentanol vollumfänglich Bezug genommen.

Hierbei kann man so vorgehen, dass man zunächst gezielt 2-Methyl-4-phenyl-2-pentanol herstellt und anschließend einer heterogen-katalytischen Hydrierung an einem zur Kernhydrierung von Aromaten geeigneten Kontakt in Analogie zu dem zuvor beschriebenen Schritt b) unterwirft.

Man kann allerdings auch so vorgehen, dass man zunächst eine Zusammensetzung aus 2-Methyl-4-phenyl-2-butanol und 2-Methyl-4-phenyl-2-pentanol herstellt, z.B. auf die zuvor für Schritt a) beschriebene Weise, diese Zusammensetzung einer heterogen-katalytischen Hydrierung an einem zur Kernhydrierung von Aromaten geeigneten Kontakt in Analogie zu dem zuvor beschriebenen Schritt b) unterwirft und die dabei erhaltene Zusammensetzung, die 4-Cyclohexyl-2-methyl-2-butanol und 4-Cyclohexyl-2-methyl-2-pentanol enthält, destillativ in ihre Bestandteile auftrennt.

Dementsprechend betrifft die Erfindung auch ein Verfahren zur Herstellung von 4-Cyclohexyl-2-methyl-2-pentanol, umfassend die folgenden Schritte:
a') Herstellung einer Zusammensetzung, enthaltend 2-Methyl-4-phenyl-2-butanol und 2-Methyl-4-phenyl-2-pentanol durch Umsetzung von Styrol mit Isopropanol bei einer Temperatur im Bereich von 250 bis 500 °C und unter Bedingungen, bei denen Isopropanol unter überkritischen Bedingungen vorliegt, wobei das Molverhältnis von Styrol zu Isopropanol im Bereich von 1 : unter 10 bis 1 : 0,5 liegt; vorzugsweise im Bereich von 1 : 9 bis 1 : 0,5, insbesondere im Bereich von 1 : 5 bis 1 : 0,5, insbesondere im Bereich von 1 : 4,5 bis 1 : 0,5, besonders bevorzugt im Bereich von 1 : 4 bis 1 : 0,75 und speziell im Bereich von 1 : 3,5 bis 1 : 1,5 insbesondere 1 : 2 bis 1 : 1 liegt.
b') heterogen-katalytische Hydrierung der in Schritt a) erhaltenen Zusammensetzung an einem zur Kernhydrierung von Aromaten geeigneten Kontakt, und
c) destillative Aufarbeitung der in Schritt b') erhaltenen Zusammensetzung, wobei man eine Zusammensetzung erhält, die im wesentlichen, d.h. zu wenigsten 90 Gew.-%, insbesondere zu wenigstens 95 Gew.-% aus 4-Cyclohexyl-2-methyl-2-pentanol besteht.

Dementsprechend betrifft die Erfindung weiterhin ein Verfahren zur Herstellung von 4-Cyclohexyl-2-methyl-2-pentanol, umfassend die folgenden Schritte:
a') Herstellung einer Zusammensetzung, enthaltend 2-Methyl-4-phenyl-2-butanol und 2-Methyl-4-phenyl-2-pentanol durch Umsetzung von Styrol mit Isopropanol bei einer Temperatur im Bereich von 250 bis 500 °C und unter Bedingungen, bei denen Isopropanol unter überkritischen Bedingungen vorliegt, wobei das Molverhältnis von Styrol zu Isopropanol im Bereich von 1 : unter 10 bis 1 : 0,5 liegt; vorzugsweise im Bereich von 1 : 9 bis 1 : 0,5, insbesondere im Bereich von 1 : 5 bis 1 : 0,5, insbesondere im Bereich von 1 : 4,5 bis 1 : 0,5, besonders bevorzugt im Bereich von 1 : 4 bis 1 : 0,75 und speziell im Bereich von 1 : 3,5 bis 1 : 1,5 insbesondere 1 : 2 bis 1 : 1 liegt.
c') destillative Aufarbeitung der in Schritt a') erhaltenen Zusammensetzung, wobei man eine Zusammensetzung erhält, die überwiegend aus 2-Methyl-4-phenyl-2-pentanol besteht,
b') heterogen-katalytische Hydrierung der in Schritt c') erhaltenen Zusammensetzung an einem zur Kernhydrierung von Aromaten geeigneten Kontakt; und gegebenenfalls
c") destillative Aufarbeitung der in Schritt b') erhaltenen Zusammensetzung, wobei man einen Zusammensetzung erhält, die im Wesentlichen aus 4-Cyclohexyl-2-methyl-2-pentanol besteht.

Es versteht sich, dass Schritt a') in Analogie zu dem zuvor bereits beschriebenen Schritt a) durchgeführt wird. Insoweit wird auf die zu Schritt a) gemachten Ausführungen für Schritt a') vollumfänglich Bezug genommen.

Es versteht sich weiterhin, dass Schritt b') in Analogie zu dem zuvor bereits beschriebenen Schritt ab) durchgeführt wird. Insoweit wird auf die zu Schritt b) gemachten Ausführungen für Schritt b') vollumfänglich Bezug genommen.

Die destillativen Schritte c), c') und c") können in Analogie zu üblichen Verfahren zur fraktionierenden Destillation durchgeführt werden. Geeignete Vorrichtungen hierfür sind dem Fachmann geläufig. Die notwendigen Bedingungen können durch Routineexperimente ermittelt werden. In der Regel erfolgt die Destillation bei vermindertem Druck.

Weiterhin betrifft die Erfindung weiterhin ein Verfahren zur Herstellung von 4-Cyclohexyl-2-methyl-2-pentanol, umfassend die folgenden Schritte:
a") Umsetzung von α-Methylstyrol mit Isopropanol bei einer Temperatur im Bereich von 250 bis 500 °C und unter Bedingungen, bei denen Isopropanol im überkritischen Zustand vorliegt, wobei man 2-Methyl-4-phenyl-2-pentanol erhält, und
b") heterogen-katalytische Hydrierung von 2-Methyl-4-phenyl-2-pentanol an einem zur Kernhydrierung von Aromaten geeigneten Kontakt,
wobei das Molverhältnis des in Schritt a") eingesetzten α-Methylstyrol zu dem in Schritt a") eingesetzten Isopropanol im Bereich von 1 : unter 5 bis 1 : 0,5 liegt; vorzugsweise im Bereich von 1 : 4,9 bis 1 : 0,5, insbesondere im Bereich von 1 : 4,5 bis 1 : 0,5, besonders bevorzugt im Bereich von 1 : 4 bis 1 : 0,75 und speziell im Bereich von 1 : 3,5 bis 1 : 1,5 insbesondere 1 : 2 bis 1 : 1 liegt.

Wie bereits zuvor erläutert, wird Schritt a") in Analogie zu dem zuvor bereits beschriebenen Schritt a) durchgeführt. Insoweit wird auf die zu Schritt a) gemachten Ausführungen für Schritt a") vollumfänglich Bezug genommen.

Es versteht sich weiterhin, dass Schritt b") in Analogie zu dem zuvor bereits beschriebenen Schritt ab) durchgeführt wird. Insoweit wird auf die zu Schritt b) gemachten Ausführungen für Schritt b") vollumfänglich Bezug genommen.

Es versteht sich weiterhin, dass das in Schritt a") erhältliche Reaktionsprodukt vor seinem Einsatz in Schritt b") in Analogie zu der zuvor für das in Schritt a) anfallende Reaktionsprodukt eine Aufarbeitung unterworfen werden kann, insbesondere einer destillativen Aufarbeitung, bei der man Leichtsieder und Schwersieder abtrennt und 2-Methyl-4-phenyl-2-pentanol als Mittelsieder gewinnt. Diesbezüglich wird ebenfalls auf die zuvor gemachten Ausführungen Bezug genommen.

### Herstellungsbeispiel 1: Herstellung des Hydrierkatalysators

Als Trägermaterial diente ein kugelförmiger SiO₂-Träger (Typ AF125 der BASF SE) mit einem Kugeldurchmesser von 3 bis 5 mm und einer Rütteldichte von 0,49 kg/l. Die BET-Oberfläche lag bei 337 m²/g, die Wasseraufnahme (WA) bei 0,83 ml/g. Zum Imprägnieren setze man eine 14,25 gew.-%ige Ruthenium(III)acetat-Lösung in Essigsäure von Umicore ein.

200 g Träger wurden in einem Rundkolben vorgelegt. 15 g der Rutheniumacetat-Lösung wurden mit destilliertem Wasser auf 150 ml verdünnt (90 % WA). Das Trägermaterial wurde im Destillationskolben eines Rotationsverdampfers vorgelegt, und das erste Viertel der Lösung wurde mit einem leichten Vakuum bei 3 bis 6 U/min auf das Trägermaterial gepumpt. Nach Beendigung der Zugabe wurde der Träger im Rotationsverdampfer weitere 10 Minuten bei 3 bis 6 U/min belassen, um den Katalysator zu homogenisieren. Dieser Tränkungs-Homogenisierungs-Schritt wurde dreimal wiederholt, bis die gesamte Lösung auf den Träger aufgebracht worden war. Das so behandelte Trägermaterial wurde im Drehrohrofen bei 140 °C bewegt getrocknet, anschließend 3 h bei 200 °C im Wasserstoffstrom reduziert (20 l/h H₂; 10 l/h N₂) und bei 25 °C passiviert (5 % Luft in N₂, 2 h). Der so erhaltene erfindungsgemäße Katalysator A enthielt 0,34 Gew. -% Ruthenium, bezogen auf das Katalysatorgewicht.

### Beispiel 1

### Schritt a)

Die Umsetzung wurde in einer kontinuierlich betriebenen Laboranlage betrieben, die als Reaktor einen 300 ml Autoklaven umfasste, der druckgeregelt betrieben wurde. So entsprach die Entnahmemenge zu jeder Zeit der Eintrittsmenge. Das entnommene Reaktionsgemisch wurde abgekühlt, entspannt und in einem Austragsbehälter gesammelt.

Eine Lösung von Styrol in Isopropanol (30 Gew.-%, 200 g/h) wurde bei einer mittleren Temperatur von 410 °C im Reaktor durch die Laboranlage gepumpt. Der Umsatz an Styrol lag bei 85,1 % und man erhielt im stationären Zustand 12,5 g/h 2-Methyl-4-phenyl-2-butanol. Die Proben wurden mittels Gaschromatographie analysiert.

### Schritt b)

In einem 300 mL-Autoklaven wurden 10,2 g 2-Methyl-4-phenyl-2-butanol (62 mmol) aus Schritt a), gelöst in 150 mL Tetrahydrofuran und 1,7 g des Katalysators aus Herstellungsbeispiel 1 in einem Katalysatorkorb vorgelegt. Der Autoklav wurde dreimal mit Stickstoff gespült und danach wurden 12 Stunden bei 200 °C und 200 bar Wasserstoffdruck aufgepresst. Nach 6 und 12 Stunden wurde der Reaktionsfortschritt mittels Gaschromatographie analysiert (30 m Säulenmaterial DB1, Innendurchmesser: 0,25 mm, Filmdicke: 0,25 µm, Temperaturprogramm 50 °C - 5 min isotherm; 6 °C / min. → 290 °C - 219 min. isotherm). Die Produktanteile sind in der nachfolgenden Tabelle angegeben.

| | 4-Cyclohexyl-2-methyl-2-butanol | 2-Methyl-4-phenyl-2-butanol | 4-Cylohexyl-2-methylbutan |
|---|---|---|---|
| 6 Stunden | 88,9% | 0% | 5,9% |
| 12 Stunden | 88,2% | 0% | 6,8% |

Nach 6 h war bereits kein Ausgangsmaterial mehr zu detektieren. Der geringe Anteil von Nebenprodukten wie 4-Cyclohexyl-2-methylbutan belegt die hohe Selektivität der Hydrierung bezüglich der gewünschten Zielverbindung 4-Cyclohexyl-2-methyl-2-butanol.

### Schritt b)

In einem 300 ml-Autoklaven wurden 100 g eines Austrages aus Schritt a), der auf einen 2-Methyl-4-phenyl-2-butanol-Gehalt von 50% auf konzentriert war und 5 g des Katalysators gemäß EP1317959 Beispiel 1 B vorgelegt. Der Autoklav wurde dreimal mit Stickstoff gespült und danach wurden 12 Stunden bei 40 °C und 150 bar Wasserstoffdruck hydriert. Nach 12 Stunden wurde der Austrag mittels Gaschromatographie analysiert (30 m Säulenmaterial DB1, Innendurchmesser: 0,25 mm, Filmdicke: 0,25 µm, Temperaturprogramm 50 °C - 5 min isotherm; 6 °C / min. · 290 °C - 219 min. isotherm). Der Umsatz von Methyl-4-phenyl-2-butanol lag bei >99,9%, die Selektivität für 4-Cyclohexyl-2-methyl-2-butanol lag bei 97,7%.

## Patentansprüche

1. Verfahren zur Herstellung von 4-Cyclohexyl-2-methyl-2-butanol, umfassend:
a) Umsetzung von Styrol mit Isopropanol bei einer Temperatur im Bereich von 250 bis 500 °C und unter Bedingungen, bei denen Isopropanol im überkritischen Zustand vorliegt, wobei man 2-Methyl-4-phenyl-2-butanol erhält, und
b) heterogen-katalytische Hydrierung von 2-Methyl-4-phenyl-2-butanol an einem zur Kernhydrierung von Aromaten geeigneten Kontakt,
wobei das Molverhältnis des in Schritt a) eingesetzten Styrols zu dem in Schritt a) eingesetzten Isopropanol im Bereich von 1 : unter 5 bis 1 : 0,5 liegt.

2. Verfahren nach Anspruch 1, wobei die Umsetzung in Schritt a) in Abwesenheit eines Katalysators durchgeführt wird.

3. Verfahren nach einem der vorhergehenden Ansprüche, wobei zur Umsetzung in Schritt a) im Wesentlichen keine von Styrol und Isopropanol verschiedenen Einsatzstoffe eingesetzt werden.

4. Verfahren nach einem der vorhergehenden Ansprüche, wobei die Umsetzung in Schritt a) bei einer Temperatur von 390 °C bis 500 °C erfolgt.

5. Verfahren nach einem der vorhergehenden Ansprüche, wobei die Umsetzung in Schritt a) bei einem Druck im Bereich von 5 bis 50 MPa erfolgt.

6. Verfahren nach einem der vorhergehenden Ansprüche, wobei man wenigstens 80 % des Isopropanols vorlegt und wenigstens 80 % des in Schritt a) eingesetzten Styrols der Umsetzung in Schritt a) unter Reaktionsbedingungen zuführt.

7. Verfahren nach einem der vorhergehenden Ansprüche, wobei man das in Schritt a) erhaltene Reaktionsgemisch, gegebenenfalls nach Entfernung von Isopropanol, unmittelbar dem Schritt b) zuführt.

8. Verfahren nach einem der Ansprüche 1 bis 7, wobei man das in Schritt a) erhaltene Reaktionsgemisch einer destillativen Aufreinigung unterwirft und das aufgereinigte 2-Methyl-4-phenyl-2-butanol dem Schritt b) zuführt.

9. Verfahren nach einem der vorhergehenden Ansprüche, wobei der in Schritt b) eingesetzte Katalysator als Aktivmetall Palladium, Platin, Eisen, Cobalt, Nickel, Rhodium, Iridium, Ruthenium, alleine oder zusammen mit mindestens einem weiteren Aktivmetall der Nebengruppen IB, VIIB oder VIIIB des Periodensystems der Elemente (CAS-Version) enthält.

10. Verfahren nach einem der vorhergehenden Ansprüche, wobei man Schritt b) in Rieselfahrweise durchführt.

11. Verfahren nach einem der vorhergehenden Ansprüche, wobei man Schritt b) in Suspensionsfahrweise durchführt.

12. Verfahren zur Herstellung von 2-Methyl-4-phenyl-2-pentanol, wobei man α-Methylstyrol mit Isopropanol bei einer Temperatur im Bereich von 250 bis 500 °C und unter Bedingungen, bei denen Isopropanol im überkritischen Zustand vorliegt, umsetzt, wobei das Molverhältnis des eingesetzten α-Methylstyrol zu dem eingesetzten Isopropanol im Bereich von 1 : unter 5 bis 1 : 0,5 liegt.

13. Verfahren zur Herstellung von 4-Cyclohexyl-2-methyl-2-pentanol, umfassend:
a") Umsetzung von α-Methylstyrol mit Isopropanol bei einer Temperatur im Bereich von 250 bis 500 °C und unter Bedingungen, bei denen Isopropanol im überkritischen Zustand vorliegt, wobei man 2-Methyl-4-phenyl-2-pentanol erhält, und
b") heterogen-katalytische Hydrierung von 2-Methyl-4-phenyl-2-pentanol an einem zur Kernhydrierung von Aromaten geeigneten Kontakt, wobei das Molverhältnis des in Schritt a") eingesetzten α-Methylstyrol zu dem in Schritt
a") eingesetzten Isopropanol im Bereich von 1 : unter 5 bis 1 : 0,5 liegt.

14. Verfahren zur Herstellung von 2-Methyl-4-phenyl-2-butanol, umfassend a) die Umsetzung von Styrol mit Isopropanol bei einer Temperatur im Bereich von 250 bis 500 °C und unter Bedingungen, bei denen Isopropanol im überkritischen Zustand vorliegt, wobei das Molverhältnis des in Schritt a) eingesetzten Styrols zu dem in Schritt a) eingesetzten isopropanol im Bereich von 1 : unter 5 bis 1 : 0,5 liegt.

## Claims

1. A process for preparing 4-cyclohexyl-2-methyl-2-butanol, comprising:
a) reaction of styrene with isopropanol at a temperature in the range from 250 to 500°C and under conditions under which isopropanol is in the supercritical state to obtain 2-methyl-4-phenyl-2-butanol, and
b) heterogeneously catalyzed hydrogenation of 2-methyl-4-phenyl-2-butanol over a catalyst suitable for ring hydrogenation of aromatics,
where the molar ratio of the styrene used in step a) to the isopropanol used in step a) is in the range from 1:below 5 to 1:0.5.

2. The process according to claim 1, wherein the reaction in step a) is performed in the absence of a catalyst.

3. The process according to either of the preceding claims, wherein essentially no feedstocks other than styrene and isopropanol are used for reaction in step a).

4. The process according to any of the preceding claims, wherein the reaction in step a) is effected at a temperature of 390°C to 500°C.

5. The process according to any of the preceding claims, wherein the reaction in step a) is effected at a pressure in the range from 5 to 50 MPa.

6. The process according to any of the preceding claims, wherein at least 80% of the isopropanol is initially charged and at least 80% of the styrene used in step a) is fed to the reaction in step a) under reaction conditions.

7. The process according to any of the preceding claims, wherein the reaction mixture obtained in step a), optionally after removal of isopropanol, is fed directly to step b).

8. The process according to any of claims 1 to 7, wherein the reaction mixture obtained in step a) is subjected to a distillative purification and the purified 2-methyl-4-phenyl-2-butanol is supplied to step b).

9. The process according to any of the preceding claims, wherein the catalyst used in step b) comprises, as an active metal, palladium, platinum, iron, cobalt, nickel, rhodium, iridium, ruthenium, alone or together with at least one further active metal of transition groups IB, VIIB or VIIIB of the Periodic Table of the Elements (CAS version).

10. The process according to any of the preceding claims, wherein step b) is performed in trickle mode.

11. The process according to any of the preceding claims, wherein step b) is performed in suspension mode.

12. A process for preparing 2-methyl-4-phenyl-2-pentanol, wherein α-methylstyrene is reacted with isopropanol at a temperature in the range from 250 to 500°C and under conditions under which isopropanol is in the supercritical state, where the molar ratio of the α-methylstyrene used to the isopropanol used is in the range from 1:below 5 to 1:0.5.

13. A process for preparing 4-cyclohexyl-2-methyl-2-pentanol, comprising
a") reaction of α-methylstyrene with isopropanol at a temperature in the range from 250 to 500°C and under conditions under which isopropanol is in the supercritical state to obtain 2-methyl-4-phenyl-2-pentanol, and
b") heterogeneously catalyzed hydrogenation of 2-methyl-4-phenyl-2-pentanol over a catalyst suitable for ring hydrogenation of aromatics, where the molar ratio of the α-methylstyrene used in step a") to the isopropanol used in step a") is in the range from 1:below 5 to 1:0.5.

14. A process for preparing 2-methyl-4-phenyl-2-butanol, comprising a) the reaction of styrene with isopropanol at a temperature in the range from 250 to 500°C and under conditions under which isopropanol is in the supercritical state, the molar ratio of the styrene used in step a) to the isopropanol used in step a) being in the range from 1:below 5 to 1:0.5.

## Revendications

1. Procédé pour la préparation de 4-cyclohexyl-2-méthyl-2-butanol, comprenant :
a) la transformation de styrène avec de l'isopropanol à une température dans la plage de 250 à 500°C et dans des conditions dans lesquelles l'isopropanol se trouve dans un état surcritique, du 2-méthyl-4-phényl-2-butanol étant obtenu et
b) l'hydrogénation sous catalyse hétérogène de 2-méthyl-4-phényl-2-butanol sur un contact approprié pour l'hydrogénation de noyaux d'aromatiques,
le rapport molaire du styrène utilisé dans l'étape a) à l'isopropanol utilisé dans l'étape a) se situant dans la plage de 1:moins de 5 à 1:0,5.

2. Procédé selon la revendication 1, la transformation dans l'étape a) étant réalisée en l'absence d'un catalyseur.

3. Procédé selon l'une quelconque des revendications précédentes, n'utilisant, pour la transformation dans l'étape a), sensiblement pas de substances de départ différentes du styrène et de l'isopropanol.

4. Procédé selon l'une quelconque des revendications précédentes, la transformation dans l'étape a) ayant lieu à une température de 390 à 500°C.

5. Procédé selon l'une quelconque des revendications précédentes, la transformation dans l'étape a) ayant lieu à une pression dans une plage de 5 à 50 MPa.

6. Procédé selon l'une quelconque des revendications précédentes, au moins 80% de l'isopropanol étant disposés au préalable et au moins 80% du styrène utilisé dans l'étape a) étant alimentés dans la transformation de l'étape a) dans des conditions de réaction.

7. Procédé selon l'une quelconque des revendications précédentes, le mélange réactionnel obtenu dans l'étape a) étant alimenté directement, le cas échéant après élimination de l'isopropanol, dans l'étape b).

8. Procédé selon l'une quelconque des revendications 1 à 7, le mélange réactionnel obtenu dans l'étape a) étant soumis à une purification par distillation et le 2-méthyl-4-phényl-2-butanol purifié étant alimenté dans l'étape b).

9. Procédé selon l'une quelconque des revendications précédentes, le catalyseur utilisé dans l'étape b) contenant, comme métal actif, du palladium, du platine, du fer, du cobalt, du nickel, du rhodium, de l'iridium, du ruthénium, seuls ou ensemble avec au moins un autre métal actif des groupes secondaires IB, VIIB ou VIIIB du système périodique des éléments (version CAS).

10. Procédé selon l'une quelconque des revendications précédentes, l'étape b) étant réalisée dans un mode opératoire à ruissellement.

11. Procédé selon l'une quelconque des revendications précédentes, l'étape b) étant réalisée dans un mode opératoire en suspension.

12. Procédé pour la préparation de 2-méthyl-4-phényl-2-pentanol, de l'α-méthylstyrène étant transformé avec de l'isopropanol à une température dans la plage de 250 à 500°C et dans des conditions dans lesquelles l'isopropanol se trouve dans un état surcritique, le rapport molaire de l'α-méthylstyrène utilisé à l'isopropanol utilisé étant situé dans la plage de 1:moins de 5 à 1:0,5.

13. Procédé pour la préparation de 4-cyclohexyl-2-méthyl-2-pentanol, comprenant :
a") la transformation d'α-méthylstyrène avec de l'isopropanol à une température dans la plage de 250 à 500°C et dans des conditions dans lesquelles l'isopropanol se trouve dans un état surcritique, du 2-méthyl-4-phényl-2-pentanol étant obtenu et
b") hydrogénation sous catalyse hétérogène de 2-méthyl-4-phényl-2-pentanol sur un contact approprié pour l'hydrogénation de noyaux d'aromatiques, le rapport molaire de l'α-méthylstyrène utilisé dans l'étape a") à l'isopropanol utilisé dans l'étape a") se situant dans la plage de 1:moins de 5 à 1:0,5.

14. Procédé pour la préparation de 2-méthyl-4-phényl-2-butanol, comprenant a) la transformation de styrène avec de l'isopropanol à une température dans la plage de 250 à 500°C et dans des conditions dans lesquelles l'isopropanol se trouve dans un état surcritique, le rapport molaire du styrène utilisé dans l'étape a) à l'isopropanol utilisé dans l'étape a) étant situé dans la plage de 1:moins de 5 à 1:0,5.
